Europäisches Patentamt

European Patent Office

Office européen des brevets

(11) Publication number: **0 338 620**
**A1**

## EUROPEAN PATENT APPLICATION

(21) Application number: 89200934.1

(22) Date of filing: **13.04.89**

(51) Int. Cl.4: **C12P 21/00 , A61K 39/29 ,**
**//C12N15/00**

(30) Priority: **18.04.88 US 182556**

(43) Date of publication of application:
**25.10.89 Bulletin 89/43**

(84) Designated Contracting States:
**CH DE FR GB IT LI NL**

(71) Applicant: MERCK & CO. INC.
126, East Lincoln Avenue P.O. Box 2000
Rahway New Jersey 07065-0900(US)

(72) Inventor: Kubek, Dennis J.
1127 Vilsmeier Road
Lansdale, PA 19446(US)

(74) Representative: Hesketh, Alan, Dr.
European Patent Department Merck & Co.,
Inc. Terlings Park Eastwick Road
Harlow Essex, CM20 2QR(GB)

(54) Recovery of hepatitis B surface antigen.

(57) A liquid phase containing hepatitis B surface antigen is separated into two phases, the HBsAg-containing phase is diluted with a physiologically acceptable liquid, sterilely filtered, and concentrated and diafiltered.

EP 0 338 620 A1

## RECOVERY OF HEPATITIS B SURFACE ANTIGEN

### BACKGROUND OF THE INVENTION

The hepatitis B virus surface antigen gene (HBsAg) has been expressed in a transformed host. The expressed protein is useful as a vaccine for the treatment and prevention of hepatitis B virus-induced diseases and/or infections and in in vitro diagnostic systems. The purification of the expressed protein from either broken cell slurries or from cell supernatant liquids, however, has heretofore resulted in large loss in yield due to degradation of the antigen.

### OBJECTS OF THE INVENTION

It is, therefore, an object of the present invention to provide a method for the recovery of HBsAg in high yield. Another object is to provide a method that facilitates purification of HBsAg. These and other objects of the present invention will be apparent from the following description.

### SUMMARY OF THE INVENTION

A liquid phase with or without solids containing HBsAg is separated into two aqueous phases after which the HBsAg-containing phase is separated, diluted with a physiologically acceptable liquid, sterilely filtered, concentrated and diafiltered to produce the purified product in greater yield.

### DETAILED DESCRIPTION

The present invention relates to the recovery and purification of HBsAg from broken cell slurries or cell supernatant liquids.

According to the present invention, the foregoing objects are achieved by separating a broken cell slurry or a cell supernatant liquid into two phases by adding liquid polyethylene glycol (PEG) and dextran. Sufficient PEG is added to bring its concentration to from about 5% to about 7%, preferably about 6%, and sufficient dextran is added to bring its concentration to from about 3% to about 5%, preferably about 4%. The addition of these two ingredients in the quantities indicated results in the formation of two aqueous phases with the HBsAg migrating to the upper more hydrophobic PEG-containing layer. A nonionic surfactant preferably is present to increase the solubility of the antigen.

The molecular weight of the PEG may vary form about 1,500 to about 5,000 and preferably is about 3350. A suitable dextran is Dextran T500 (500,000 mw). An example of a nonionic surfactant is polyoxyelthylene (9) octaphenol (Triton X-100) which is used in an amout up to about 0.3%, preferably about 0.2%.

The broken cell slurry or cell supernatant liquid with the foregoing added ingredients is centrifuged for from about 5 to about 30 minutes at from about 1000xg to about 4000xg at a temperature of from about 2 to about 8° C. The supernatant liquid then is drawn off, diluted with from about 1 to about 3 volumes of a physiologically acceptable liquid, e.g., phosphate buffered saline (PBS), physiological saline or water, and sterile filtered.

The filtrate is concentrated and diafiltered to remove PEG and residual surfactant is removed by resin treatment.

The following example illustrates the present invention without, however, limiting the same thereto.

### EXAMPLE 1

Yeast cells that had been transformed with a vector constructed to express HBsAg, as described in published European patent application 0 020 251 (the disclosure of which is hereby incorporated by reference), were grown in YEHD medium to stationary phase, after which the yeast cells were broken.

To a 790 ml slurry of broken yeast cells containing 0.2% of Triton X-100 there were added 480 ml of a 20% solution of PEG 3350. After mixing, 320 ml of a 20% solution of Dextran T500 were added and mixed. The resulting mixture was centrifuged at 2500 x g at 4° C for 15 minutes after which the HBsAg was contained in the more hydrophobic PEG 3350 supernatant fluid. This phase was separated, diluted with 2 volumes of PBS and sterile filtered through a 0.2 μ filter. The filtrate was concentrated and diafiltered with 10 volumes PBS on an Amicon $10^5$ mw cutoff membrane to remove the PEG 3350 . Residual Triton X-100 was removed with a polystyrene divinylbenzene resin (XAD-2) and the resin beads were then separated by filtration.

The ratio of recovered HBsAg to total protein was 23.6% compared to a ratio of only 7.8% for a production process involving adsorption to fused silica.

## EXAMPLE 2

Yeast cells that had been transformed with a vector constructed to express HBsAg, as described in published European patent application 0 020 251 (the disclosure of which is hereby incorporated by reference), were grown in YEHD medium to stationary phase, after which the yeast cells were broken.

To a 700 ml slurry of broken yeast cells containing 0.2% of Triton X-100 there were added 420 ml of a 20% solution of PEG 3350. After mixing, 280 ml of a 20% solution of Dextran T500 were added and mixed. The resulting mixture was centrifuged at 2500 x g at 4°C for 15 minutes after which the HBsAg was contained in the more hydrophobic PEG 3350 supernatant fluid. This phase was separated, diluted with 2 volumes of PBS and sterile filtered through a 0.2 μ filter. The filtrate was concentrated and diafiltered with 10 volumes PBS on an Amicon $10^5$ mw cutoff membrane to remove the PEG 3350 . Residual Triton X-100 was removed with a polystyrene divinylbenzene resin (XAD-2) and the resin beads were then separated by filtration.

The ratio of recovered HBsAg to total protein was 28% compared to a ratio of only 7.8% for a production process involving adsorption to fused silica.

## Claims

1. A method of isolating yeast-produced HBsAg from a broken cell slurry or a cell culture supernatant liquid comprising:
adding to the cell slurry or supernatant liquid a quantity of PEG and dextran sufficient to form an upper hydrophobic layer and a lower hydrophoilic layer, centrifuging the resulting mixture, diluting the supernatant liquid with a physiologically acceptable liquid, and concentrating and diafiltering the diluted supernatant liquid.

2. A method according to claim 1 wherein the quantity of PEG is from about 5% to about 7% by volume relative to the total volume of the cell slurry or supernatant liquid mixture .

3. A method according to claim 2 wherein the quantity of PEG is about 6%.

4. A method according to claim 1 wherein the quantity of dextran is from about 3% to about 5% by volume relative to the total volume of the cell slurry or supernatant liquid mixture.

5. A method according to claim 6 wherein the dextran is present in a quantity of about 4% by volume.

6. A method according to claim 1 wherein the PEG has a molecular weight of from about 3350.

7. A method according to claim 1 wherein the dextran has a molecular weight of from about 450,000 to about 550,000.

8. A method according to claim 1 wherein the centrifuging takes from about 5 to about 30 minutes at from about 1000xg to about 4000xg at a temperature of from about 2 to about 8°C.

9. A method according to claim 8 wherein the centrifuging is carried out at about 15 minutes at 2500xg at 4°C.

10. A method according to claim 1 wherein the physiologically acceptable liquid is PBS, saline or water.

11. A method according to claim 10 wherein the quantity of the pbysiologically acceptable liquid is from about 1 to about 3 volumes relative to the volume of the supernatant liquid.

12. A method according to claim 1 wherein a nonionic surfactant is added to the cell slurry or supernatant liquid in a quantity of from about 0.1% to about 0.3%.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | NUCLEIC ACIDS RESEARCH, vol. 11, no. 9, 1983, pages 2745-2763, IRL Press Ltd, Oxford, GB; R.A. HITZEMAN et al.: "Expression of hepatitis B virus surface antigen in yeast" * Page 2748, lines 10-29; page 2757, line 9 - page 2758, line 8 * | 1-12 | C 12 P 21/00 A 61 K 39/29 // C 12 N 15/00 |
| A | PROC. NATL. SCI. USA, vol. 80, January 1983, pages 1-5; A. MIYANOHARA et al.: "Expression of hepatitis B surface antigen gene in yeast" | | |
| D,A | EP-A-0 020 251 (THE REGENTS OF THE UNIVERSITY OF CALIFORNIA) | | |

TECHNICAL FIELDS SEARCHED (Int. Cl.4)

A 61 K
C 12 P
C 12 N

The present search report has been drawn up for all claims

| Place of search | Date of completion of the search | Examiner |
|---|---|---|
| THE HAGUE | 12-07-1989 | SKELLY J.M. |